(19) **Europäisches Patentamt**
**European Patent Office**
**Office européen des brevets**

(11) **EP 3 781 040 B1**

(12) **FASCICULE DE BREVET EUROPEEN**

(45) Date de publication et mention
de la délivrance du brevet:
**11.12.2024 Bulletin 2024/50**

(21) Numéro de dépôt: **19722797.8**

(22) Date de dépôt: **16.04.2019**

(51) Classification Internationale des Brevets (IPC):
**A61B 8/08** *(2006.01)*   **A61B 5/00** *(2006.01)*
**G16H 50/30** *(2018.01)*

(52) Classification Coopérative des Brevets (CPC):
**A61B 8/0875; A61B 5/4542; A61B 8/0858;**
**A61B 8/5223; G16H 50/30;** A61B 5/4504

(86) Numéro de dépôt international:
**PCT/EP2019/059739**

(87) Numéro de publication internationale:
**WO 2019/201888 (24.10.2019 Gazette 2019/43)**

(54) **DISPOSITIF ET PROCEDE DE CONTROLE DE LA STABILITE D'UN IMPLANT DENTAIRE**

VORRICHTUNG UND VERFAHREN ZUR KONTROLLE DER STABILITÄT EINES
ZAHNIMPLANTATS

DEVICE AND METHOD FOR CONTROLLING THE STABILITY OF A DENTAL IMPLANT

(84) Etats contractants désignés:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priorité: **16.04.2018 FR 1853306**

(43) Date de publication de la demande:
**24.02.2021 Bulletin 2021/08**

(73) Titulaires:
• **Centre national de la recherche scientifique**
**75016 Paris (FR)**
• **Assistance Publique - Hôpitaux de Paris**
**75004 Paris (FR)**
• **Université Paris XII Val de Marne**
**94010 Créteil Cedex (FR)**

(72) Inventeurs:
• **HAIAT, Guillaume**
**94150 RUNGIS (FR)**
• **VAYRON, Romain**
**59300 Valenciennes (FR)**

(74) Mandataire: **Hautier IP**
**20, rue de la Liberté**
**06000 Nice (FR)**

(56) Documents cités:
**EP-A1- 2 503 954        EP-A1- 2 503 954**

EP-B1- 2 503 954        US-A1- 2012 244 489
US-A1- 2012 244 489     US-A1- 2013 122 458
US-A1- 2013 122 458

• **HAMED HAMID MUHAMMED ET AL: "Using**
**Ultrasonic Spectrometry to Estimate the Stability**
**of a Dental Implant Phantom", ENGINEERING**
**(ENG ), vol. 05, no. 10, 1 January 2013**
**(2013-01-01), pages 570 - 574, XP055530353,**
**ISSN: 1947-3931, DOI: 10.4236/eng.2013.510B117**
• **ROMAIN VAYRON ET AL: "Assessment of In Vitro**
**Dental Implant Primary Stability Using an**
**Ultrasonic Method", ULTRASOUND IN MEDICINE**
**AND BIOLOGY., vol. 40, no. 12, 1 December 2014**
**(2014-12-01), US, pages 2885 - 2894,**
**XP055530349, ISSN: 0301-5629, DOI:**
**10.1016/j.ultrasmedbio.2014.03.035**
• **ROMAIN VAYRON ET AL: "Comparison of**
**Resonance Frequency Analysis and of**
**Quantitative Ultrasound to Assess Dental Implant**
**Osseointegration", SENSORS, vol. 18, no. 5, 2**
**May 2018 (2018-05-02), pages 1397,**
**XP055530340, DOI: 10.3390/s18051397**
• **ROMAIN VAYRON ET AL: "Assessment of In Vitro**
**Dental Implant Primary Stability Using an**
**Ultrasonic Method", ULTRASOUND IN MEDICINE**
**AND BIOLOGY., vol. 40, no. 12, 1 December 2014**
**(2014-12-01), US, pages 2885 - 2894,**
**XP055530349, ISSN: 0301-5629, DOI:**
**10.1016/j.ultrasmedbio.2014.03.035**

- ROMAIN VAYRON ET AL: "Comparison of Resonance Frequency Analysis and of Quantitative Ultrasound to Assess Dental Implant Osseointegration", SENSORS, vol. 18, no. 5, 2 May 2018 (2018-05-02), pages 1397, XP055530340, DOI: 10.3390/s18051397
- HAMED HAMID MUHAMMED ET AL: "Using Ultrasonic Spectrometry to Estimate the Stability of a Dental Implant Phantom", ENGINEERING (ENG ), vol. 05, no. 10, 1 January 2013 (2013-01-01), pages 570 - 574, XP055530353, ISSN: 1947-3931, DOI: 10.4236/eng.2013.510B117

**Description**

DOMAINE TECHNIQUE

Le présent exposé concerne un dispositif et un procédé de contrôle de la stabilité d'un implant dentaire inséré au moins partiellement dans un os.

ARRIERE PLAN

Un implant dentaire se présente classiquement sous la forme d'une racine dentaire artificielle, généralement en alliage de titane, mise en place à l'intérieur de l'os de la mâchoire supérieure ou inférieure. Un élément de prothèse dentaire est ensuite vissé dans l'implant. Souvent, après la pose de l'implant dentaire, une période de cicatrisation est nécessaire pour que les cellules osseuses colonisent la surface enfouie de l'implant et que celui-ci soit "ostéo-intégré", c'est-à-dire intégré dans l'os, sans interposition de tissu fibreux au niveau de l'interface os-implant, ou frontière, entre l'os et l'implant.

L'implant dentaire jouant le rôle d'intermédiaire entre l'élément de prothèse et l'os de la mâchoire et transmettant notamment les forces au support osseux, il doit être bien intégré dans celui-ci. Durant la période de cicatrisation précitée, l'implant est laissé au repos afin d'obtenir son intégration dans l'os, pour qu'il puisse ensuite être en mesure de supporter les charges qui s'exerceront sur lui. Après la période de cicatrisation, le chirurgien met en place l'élément de prothèse dans l'implant dentaire. La mesure de la bonne intégration de l'implant dans l'os, ou ostéo-intégration, s'avère donc primordiale pour la réussite du traitement, en particulier pour déterminer précisément à quel moment terminer la période de cicatrisation et charger l'implant avec l'élément de prothèse.

Le document de brevet EP 2503954 décrit un dispositif de contrôle de la stabilité d'un implant dentaire dans un os et un dispositif associé. Ce procédé consiste à calculer, à partir d'un échogramme, un indicateur dont la valeur permet d'évaluer l'ostéo-intégration de l'implant. Bien que cet indicateur soit satisfaisant dans des conditions particulières, il subsiste un besoin pour un indicateur permettant d'évaluer finement l'ostéo-intégration de

l'implant dans des conditions plus générales.

L'article de Romain Vayron et al., "Assessment of In Vitro Dental Implant Primary Stability Using an Ultrasonic Method", Ultrasound in Medicine & Biology, vol. 40., no. 12, pages 2885-2894, divulgue les caractéristiques du préambule de la revendication 1.

PRESENTATION GENERALE

Le présent exposé concerne un dispositif de contrôle de la stabilité d'un implant dentaire inséré au moins partiellement dans un os, l'implant ayant une extrémité libre émergeant à la surface de l'os et une extrémité enfouie dans l'os, située à l'opposé de l'extrémité libre. Ce dispositif comprend un transducteur ultrasonore adapté pour:

- être couplé directement ou indirectement à l'implant,
- émettre une onde ultrasonore se propageant à l'intérieur de l'implant vers l'extrémité enfouie,
- recueillir l'onde ultrasonore réfléchie au niveau d'une interface de contact entre l'implant et l'os, et fournir un signal de mesure représentant l'onde ultrasonore réfléchie.

Le dispositif comprend également une unité de traitement adaptée pour calculer, à partir du signal de mesure, un indicateur dont la valeur permet d'évaluer l'intégration de l'implant dans l'os.

L'indicateur proposé correspond à l'énergie moyenne du signal de mesure, sur un intervalle de temps t1-t2 débutant à un premier instant t1 et se terminant à un deuxième instant t2, le premier instant t1 étant compris entre 20 et 80 $\mu$s après le début de l'émission de l'onde ultrasonore par le transducteur. Un tel indicateur s'avère particulièrement sensible à l'ostéo-intégration de l'implant et permet donc d'évaluer finement celle-ci.

Le présent exposé concerne également un procédé de contrôle de la stabilité d'un implant dentaire inséré au moins partiellement dans un os, l'implant ayant une extrémité libre émergeant à la surface de l'os et une extrémité enfouie dans l'os, située à l'opposé de l'extrémité libre. Le procédé comprend les étapes suivantes:

- émettre une onde ultrasonore se propageant à l'intérieur de l'implant vers l'extrémité enfouie, recueillir l'onde ultrasonore réfléchie au niveau d'une interface de contact entre l'implant et l'os, et fournir un signal de mesure représentant l'onde ultrasonore réfléchie,
- calculer, à partir du signal de mesure, ledit indicateur.

Bien entendu, le dispositif précité peut être utilisé pour mettre en oeuvre le procédé.

Outre les caractéristiques mentionnées plus haut, le dispositif et le procédé proposés peuvent présenter une ou plusieurs des caractéristiques parmi les suivantes, considérées isolément ou selon des combinaisons techniquement possibles.

- Le premier instant t1 est compris entre 30 et 60 $\mu$s. Ceci permet d'améliorer encore la sensibilité de l'indicateur.
- Le deuxième instant t2 est, au minimum, séparé du premier instant t1 de 5 $\mu$s (i.e. $t2 \geq t1 + 5$ $\mu$s) et, au maximum, égal à 200 $\mu$s.
- Le transducteur ultrasonore est adapté pour émettre une onde ultrasonore dont la fréquence centrale est au moins égale à 5 MHz. En dessous de cette valeur, le signal de mesure recueilli est moins représentatif de l'interface os-implant et, de ce fait, l'indicateur calculé caractérise moins bien cette interface. En particulier, la fréquence centrale peut être au moins égale à 8 MHz et, par exemple, environ égale à 10 Mhz. Les transducteurs ultrasonores ayant une fréquence centrale de 10 MHz se trouvent facilement dans le commerce, à moindre coût, et cette fréquence se révèle bien adaptée à l'application envisagée.
- Le transducteur ultrasonore a une surface active adaptée pour être au contact de l'extrémité libre de l'implant, directement ou via une pièce intermédiaire, parallèlement à une surface d'appui définie par l'extrémité libre de l'implant, de manière à émettre une onde ultrasonore se propageant à l'intérieur de l'implant selon une direction de propagation perpendiculaire à la surface active du transducteur.
- L'unité de traitement comprend des moyens d'amplification pour amplifier le signal de mesure, avant de calculer l'indicateur, le signal de mesure étant amplifié avec un gain ajusté de manière à obtenir une saturation du signal jusqu'au premier instant mais pas au delà. Par ailleurs, le gain peut être variable en fonction du temps et augmenter, par exemple de manière continue ou par paliers successifs, entre les premier et deuxième instants. Ceci permet d'augmenter la taille de l'intervalle de temps t1-t2 sans obtenir de saturation du signal, et donc de gagner en sensibilité.

Les caractéristiques et avantages précités, ainsi que d'autres, apparaîtront à la lecture de la description détaillée qui suit, d'exemples de réalisation du dispositif proposé. Cette description détaillée fait référence aux dessins annexés.

BREVE DESCRIPTION DES DESSINS

Les dessins annexés sont schématiques et ne sont pas à l'échelle, ils visent avant tout à illustrer les principes de l'invention.

La FIG 1 est une représentation schématique d'ensemble d'un exemple de dispositif de contrôle.

La FIG 2 représente le spectre d'émission du signal correspondant à l'onde ultrasonore utilisée.

La FIG 3 est un exemple de signal de mesure obtenu au moyen du dispositif de la FIG 1.

La FIG 4 est une photographie de quatre implants dentaires 1 identiques, insérés dans un bloc de résine avec différents niveaux d'enfoncement.

La FIG 5 est un graphique représentant la variation du gain appliqué au signal de mesure, exprimé en dB, en fonction de t1, exprimé en centièmes de $\mu$s (i.e. $10^{-8}$s).

La FIG 6 est un graphique représentant la variation de la sensibilité S, exprimée en pourcentage, en fonction de t1, exprimé en centièmes de $\mu$s (i.e. $10^{-8}$ s).

DESCRIPTION DETAILLEE D'EXEMPLE(S)

Des exemples de réalisation sont décrits en détail ci-après, en référence aux dessins annexés. Ces exemples illustrent les caractéristiques et les avantages de l'invention. Il est toutefois rappelé que l'invention ne se limite pas à ces exemples.

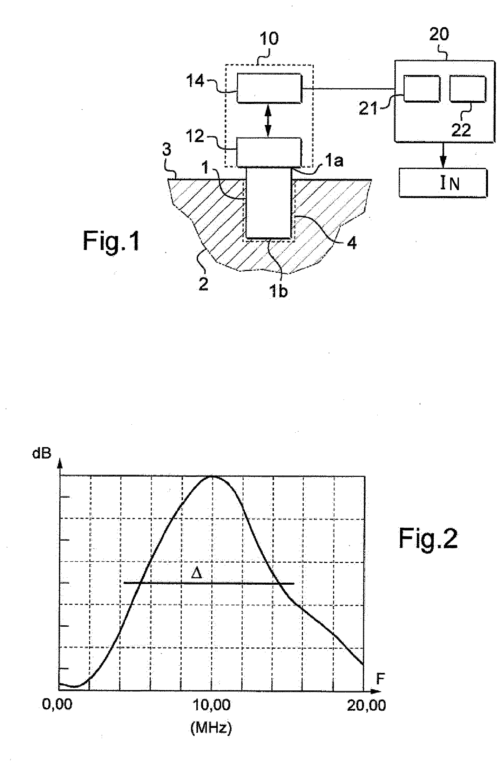

La FIG 1 représente un exemple de dispositif de contrôle de la stabilité d'un implant dentaire inséré dans un os 2. L'implant dentaire 1 est représenté schématiquement sous la forme d'un cylindre. En réalité, cependant, un tel implant 1 présente une forme effilée et un filetage extérieur, de manière à pouvoir être vissé dans l'os 2. Par exemple, l'implant présente un diamètre moyen de 2.5 à 5.5 mm de diamètre et une longueur de 6 à 16 mm. L'implant dentaire est, typiquement, en alliage de titane. Une fois ancré dans l'os 2, l'implant 1 présente une extrémité libre 1a émergeant à la surface 3 de l'os 2, à l'opposé d'une extrémité 1b enfouie dans l'os 2, de sorte que l'extrémité libre 1a de l'implant 1 est susceptible d'être accessible in vivo.

Le dispositif de contrôle comprend principalement un capteur ultrasonore 10 et une unité de calcul 20. Le capteur ultrasonore 10, de type piézo-électrique, comprend un transducteur ultrasonore 12 pour émettre une onde ultrasonore en régime impulsionnel et recevoir les échos résultant de la réflexion de l'onde ultrasonore se propageant dans l'implant, et un circuit de commande 14 pour commander le transducteur ultrasonore 12. Le transducteur ultrasonore 12 est prévu pour être utilisé en mode échographique et placé de sorte que sa surface active soit couplée, directement ou indirectement (i.e. au moyen d'une pièce intermédiaire, non représentée), avec l'extrémité libre 1a de l'implant, et de sorte que cette surface active soit sensiblement parallèle à la surface d'appui circulaire définie par l'extrémité libre 1a de l'implant. L'extrémité libre de l'implant 1 est ainsi utilisée comme site d'accueil pour positionner le transducteur 12 ou la pièce intermédiaire.

Lorsqu'une pièce intermédiaire (non représentée) est utilisée pour coupler le transducteur 12 à l'implant 1, elle est adaptée pour transmettre l'onde ultrasonore entre le transducteur 12 et l'implant 1. Pour permettre cette transmission, la pièce intermédiaire peut être fixée mécaniquement, d'un côté, sur la surface active du transducteur 12 et, de l'autre, à l'implant 1. Elle est fixée sur la surface active du transducteur 12, par exemple, par collage, vissage ou sertissage. Elle est fixée à l'implant 1, par exemple, par vissage à l'intérieur d'une cavité ménagée dans l'implant 1. Une telle cavité (non représentée) débouche à l'extrémité libre 1a de l'implant et peut s'étendre plus ou moins profondément à l'intérieur de l'implant 1 en direction de son extrémité enfouie 1b.

Dans ces conditions, l'onde ultrasonore émise par le transducteur 12 peut se propager à l'intérieur de l'implant 1, perpendiculairement à la surface active du transducteur 12, depuis l'extrémité libre 1a de l'implant vers son extrémité enfouie 1 b, tout en favorisant le phénomène d'interaction de l'onde ultrasonore, qui se propage dans l'implant, avec une interface de contact os-implant 4 située à la frontière entre la surface externe de l'implant et l'os 2 entourant celui-ci. Ce phénomène d'interaction est essentiel, comme il apparaîtra par la suite.

Le circuit de commande 14 comprend un générateur d'impulsions électriques utilisé pour exciter l'élément piézo-électrique du transducteur 12, lequel transforme les impulsions électriques reçues en une onde ultrasonore correspondante, qui se propage ensuite dans l'implant 1 vers l'extrémité enfouie 1b. Le générateur d'impulsions électriques produit, par exemple, des signaux du type impulsion brève ou signal carré à fronts de montée et de descente rapides. Le circuit de commande 14 comprend également un circuit de réception recevant le signal électrique, ou signal de mesure, délivré par le transducteur ultrasonore 12 et correspondant aux à l'onde ultrasonore réfléchie au niveau de l'interface de contact 4 et reçues par le transducteur 12.

L'unité de calcul 20, reliée au capteur 10, par exemple par l'intermédiaire d'une ligne de transmission de type coaxiale, comprend principalement une mémoire informatique 21 pour mémoriser le signal électrique représentatif de l'onde ultrasonore réfléchie, recueillie par le circuit de réception du circuit de commande, et une unité de traitement 22 configurée pour traiter le signal mémorisé, comme détaillé par la suite. Cette unité de calcul 20 peut être une unité indépendante de type microcontrôleur ou un ordinateur individuel. Divers équipements peuvent être ajoutés, tels qu'un moyen d'affichage et un moyen d'impression.

Compte tenu de l'application visée, la fréquence centrale de l'onde ultrasonore émise est de préférence choisie au moins égale à 5 MHz, en particulier au moins égale à 8 MHz. La bande passante mesurée à -6 dB peut être de l'ordre de 80% de la fréquence centrale. La limite inférieure de la bande passante peut être supérieure ou égale à 30 MHz. La FIG 2 montre un spectre d'émission du signal correspondant à une onde ultrasonore émise avec une fréquence centrale égale à 10 MHz et une bande passante A comprise entre 6 et 14 MHz.

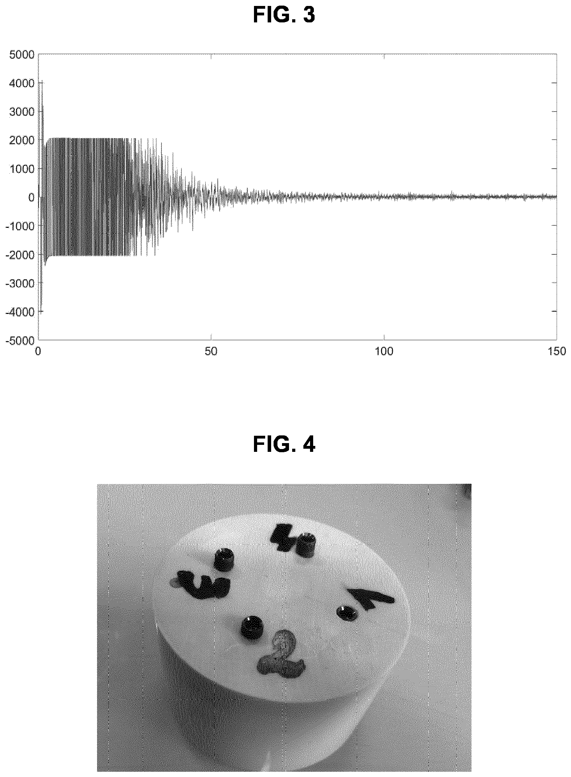

La largeur d'impulsion du signal électrique envoyé au transducteur 12 par le circuit de commande 14 est de préférence choisie inférieure ou égale à la moitié d'une période correspondant à la fréquence de résonance du transducteur ultrasonore 12. Par exemple, pour une fréquence centrale égale à 10 MHz, la largeur d'impulsion est de 50 ns. Un exemple de signal de mesure, ou échogramme, obtenu lors du contrôle de la stabilité d'un implant est représenté sur la FIG 3. L'amplitude du signal, en ordonnée, est donnée en unités arbitraires. Le temps, en abscisse, est donné en $\mu$s (i.e. $10^{-6}$ s). Dans cet exemple, le signal a été amplifié avec un gain de 70 dB, de manière à obtenir une saturation du signal jusqu'à t1 = 40 $\mu$s, le signal n'étant plus saturé ensuite.

Le traitement du signal de mesure représentant l'onde ultrasonore réfléchie peut comprendre plusieurs étapes, mais consiste pour l'essentiel à calculer un indicateur IN dont la valeur permet d'évaluer l'intégration de l'implant dans l'os.

Cet indicateur IN correspond à l'énergie moyenne du signal de mesure, sur un intervalle de temps t1-t2 débutant à un instant t1 et se terminant à un instant t2.

L'énergie moyenne peut être déterminée en calculant l'intégrale de la valeur absolue de l'amplitude du signal au carré, sur l'intervalle de temps t1-t2. Dans ce cas, on peut avoir:

$$IN = \int_{t_1}^{t_2} |M(t)|^2 dt$$

où M(t) désigne le signal de mesure.

L'énergie moyenne peut cependant être déterminée différemment, sans sortir du cadre de l'invention. Par exemple, l'indicateur IN peut être calculé selon la formule suivante:

$$IN = \int_{t_1}^{t_2} |M(t)|\, dt$$

Au lieu de considérer le carré de l'amplitude du signal, ou la valeur absolue de l'amplitude du signal, on peut également considérer l'enveloppe E(t) du signal de mesure ou toute autre grandeur physique liée à l'énergie contenue dans le signal, dans la fenêtre temporelle considérée.

Dans leurs recherches ayant conduit à l'invention, les inventeurs ont découvert qu'il existait une valeur optimale pour le choix de l'instant t1 permettant d'optimiser la sensibilité de l'indicateur et, ainsi, d'évaluer plus finement l'ostéo-intégration de l'implant. De manière surprenante, des valeurs relativement élevées doivent être choisies pour t1. Cette découverte est illustrée par la série d'expériences suivantes.

(Matériel et méthode)

Les expériences ont été réalisées à l'aide d'une sonde ultrasonore du type de celle de la FIG 1 mais équipée d'une pièce intermédiaire entre le transducteur 12 et l'implant 1. Quatre implants dentaires 1 strictement identiques ont été insérés dans un bloc de résine avec différents niveaux d'enfoncement, comme le montre la photographie de la FIG 4.

Les implants sont numérotés dans l'ordre d'un enfoncement décroissant. Les implants étant plus ou moins enfoncés, leur stabilité est plus ou moins importante. Ainsi, l'implant numéro 1, totalement enfoncé dans la résine, est très stable. Sa stabilité correspond à celle d'un implant parfaitement ostéo-intégré. Inversement, l'implant numéro 4, à peine enfoncé dans la résine, est très instable. Sa stabilité correspond à celle d'un implant qui ne serait pas du tout ou très peu ostéo-intégré.

Les implants numéro 2 et 3 correspondent respectivement à un implant relativement stable et relativement instable. Les résultats obtenus avec les implants numéro 2 et 3 ont été comparés pour évaluer la sensibilité de l'indicateur IN.

Le calcul de l'indicateur IN s'effectue dans une fenêtre temporelle comprise entre t1 et t2, étant entendu que le deuxième instant t2 est postérieur au premier instant t1, soit t2 > t1. De manière générale, la valeur de t2 est choisie pour obtenir un compromis acceptable entre une durée suffisante pour obtenir des informations pertinentes et le besoin d'un rapport signal/bruit satisfaisant. Ces conditions générales étant respectées, les inventeurs ont constaté que la valeur de t2 n'influait pas significativement sur la sensibilité de l'indicateur IN à partir du moment où la durée de la fenêtre t1-t2 était au moins égale à environ 5 μs. Aussi, dans les expériences réalisées, la valeur de t2 a été choisie égale à 150 μs pour l'ensemble des mesures. La valeur de t2 peut toutefois, plus généralement, être choisie entre t1+5 μs et 200 μs. Par exemple, pour t1 = 40 μs, t2 peut être choisi entre 45 et 200 μs.

Une variation de la valeur de t1 entre 5 et 100 μs a été considérée. Pour chaque valeur de t1 considérée, la même procédure a été utilisée pour ajuster le gain de façon à pouvoir visualiser le signal avec un numériseur classique. Cette procédure consiste à ajuster le gain de manière à obtenir une saturation du signal jusqu'au début de la fenêtre temporelle considérée (autour de l'instant t1 donc), le signal n'étant plus saturé ensuite. Ceci est illustré sur la FIG 3 qui représente un exemple de signal de mesure obtenu lors du contrôle de la stabilité de l'implant numéro 3. La valeur de t1 considérée est de 40 μs. Le signal a été amplifié et le gain ajusté à 70 dB de manière à obtenir une saturation du signal jusqu'à 40 μs, le signal n'étant plus saturé ensuite.

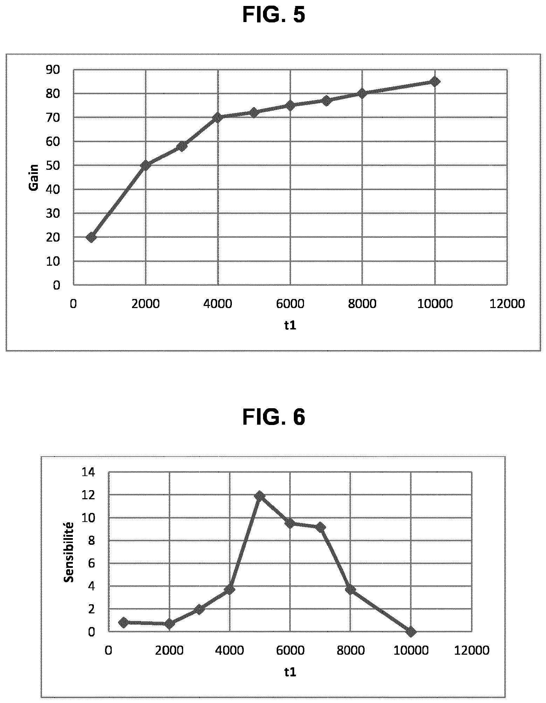

Cette procédure a été appliquée en considérant l'implant numéro 3 car il s'agit de l'implant permettant d'obtenir une valeur maximale du signal. Notons qu'il n'est pas nécessaire d'avoir un réglage très précis du gain mais que celui-ci doit être le même pour les deux implants (numéros 2 et 3) considérés et pour la même valeur de t1. La Figure 5 montre la variation du gain, exprimé en dB, en fonction de t1, exprimé en centièmes de μs (i.e. $10^{-8}$ s). Le gain augmente en fonction de t1 car plus on mesure le signal à un temps élevé, plus il est nécessaire d'amplifier le signal sachant que celui-ci diminue en fonction du temps.

Une fois la valeur du gain déterminée pour chaque valeur de t1, trois mesures ont été effectuées avec chaque implant et chaque valeur de t1, en utilisant le gain déterminé précédemment. Les résultats obtenus avec les implants numéro 2 et 3 ont été comparés et une sensibilité S(t1) de l'indicateur IN a été estimée pour chaque valeur de t1. Lorsque les valeurs de l'indicateur IN ne présentent pas de différences significatives entre les deux implants, la sensibilité S a été fixée égale à 0. Dans le cas contraire, la sensibilité S est donnée par la variation relative de la moyenne des indicateurs obtenue pour les implants 2 et 3, soit:

$$S(t1) = \frac{\big(M(t1,3) - M(t1,2)\big) * 200}{M(t1,3) + M(t1,2)}$$

où M(t1,i) correspond à la moyenne des indicateurs obtenu pour l'implant numéro i et l'indicateur calculé avec t1. La valeur de S augmente donc avec la sensibilité de l'indicateur IN.

(Résultats et conclusion)

La Figure 6 montre la variation de la sensibilité S, sans unité, en fonction de t1, exprimé en centièmes de $\mu$s (i.e. $10^{-8}$ s). De manière surprenante, une valeur optimale est obtenue pour une valeur de t1 située autour de 50 $\mu$s. La sensibilité est bonne, i.e. supérieure ou proche de 4, pour des valeurs de t1 comprises entre 40 et 80 $\mu$s.

A l'heure actuelle, il est difficile d'expliquer l'existence d'une valeur optimale pour t1 et le fait que cette valeur soit relativement élevée. L'hypothèse suivante peut toutefois être avancée.

En considérant un temps de propagation relativement long, l'onde ultrasonore effectue un grand nombre d'aller-retours dans l'implant (qui se comporte comme un guide d'onde). Par conséquent, plus le temps est important, plus l'amplitude du signal de mesure est sensible à des variations de conditions aux limites de l'implant car l'onde ultrasonore aura effectué plus d'aller-retour dans l'implant et donc interagit de façon plus conséquente avec l'interface os-implant. Par conséquent, si la valeur de t1 est trop faible, le signal utile pour le calcul de l'indicateur IN est compris dans des temps relativement faibles du fait de la décroissance exponentielle du signal, ce qui ne permet pas d'obtenir une bonne sensibilité du fait de la nature de l'interaction entre l'onde ultrasonore et l'implant. D'un autre côté, si la valeur de t1 est trop élevée, le signal devient fortement affecté par le bruit et il n'est pas possible d'obtenir une bonne sensibilité non plus.

Enfin, on notera que les expériences réalisées avec un implant dentaire inséré (au moins partiellement) dans un os concordent avec les résultats précités obtenus pour un implant inséré dans une résine. En particulier, ces expériences montrent également l'existence d'une valeur optimale pour t1 et le fait que cette valeur optimale soit relativement élevée. Toutefois, bien qu'étant relativement élevée, la valeur optimale de t1 est généralement plus faible que la valeur optimale obtenue avec la résine.

**Revendications**

1.  Dispositif de contrôle de la stabilité d'un implant dentaire (1) inséré au moins partiellement dans un os (2), l'implant ayant une extrémité libre (1a) émergeant à la surface de l'os et une extrémité enfouie (1b) dans l'os (2), située à l'opposé de l'extrémité libre, le dispositif comprenant:

    - un transducteur ultrasonore (12) adapté pour être couplé directement ou indirectement à l'implant (1), émettre une onde ultrasonore se propageant à l'intérieur de l'implant (1) vers l'extrémité enfouie (1b), recueillir l'onde ultrasonore réfléchie au niveau d'une interface de contact (4) entre l'implant (1) et l'os (2), et fournir un signal de mesure représentant l'onde ultrasonore réfléchie,
    - une unité de traitement adaptée pour calculer, à partir du signal de mesure, un indicateur (IN) dont la valeur permet d'évaluer l'intégration de l'implant (1) dans l'os (2), l'indicateur (IN) correspondant à l'énergie moyenne du signal de mesure sur un intervalle de temps (t1-t2) débutant à un premier instant (t1) et se terminant à un deuxième instant (t2), **caractérisé en ce que** le premier instant (t1) est compris entre 20 et 80 $\mu$s après le début de l'émission de l'onde ultrasonore par le transducteur (12).

2.  Dispositif selon la revendication 1, dans lequel le premier instant (t1) est compris entre 30 et 60 $\mu$s.

3.  Dispositif selon la revendication 1 ou 2, dans lequel le deuxième instant (t2) est, au minimum, séparé du premier instant (t1) de 5 $\mu$s et, au maximum, égal à 200 $\mu$s.

4.  Dispositif selon l'une quelconque des revendications 1 à 3, dans lequel le transducteur ultrasonore est adapté pour émettre une onde ultrasonore dont la fréquence centrale est au moins égale à 5 MHz et, en particulier, au moins égale 8 MHz.

5.  Dispositif selon l'une quelconque des revendications 1 à 4, dans lequel le transducteur ultrasonore (12) a une surface active adaptée pour être au contact de l'extrémité libre (1a) de l'implant, directement ou via une pièce intermédiaire, parallèlement à une surface d'appui définie par l'extrémité libre de l'implant, de manière à émettre une onde ultrasonore se propageant à l'intérieur de l'implant (1) selon une direction de propagation perpendiculaire à la surface active du transducteur (12).

6.  Dispositif selon l'une quelconque des revendications 1 à 5, dans lequel l'unité de traitement comprend des moyens d'amplification pour amplifier le signal de mesure avant de calculer l'indicateur (IN), le signal de mesure étant amplifié avec un gain ajusté de manière à obtenir une saturation du signal jusqu'au premier instant (t1) mais pas au delà.

7. Dispositif selon l'une quelconque des revendications 1 à 6, dans lequel l'unité de traitement comprend des moyens d'amplification pour amplifier le signal de mesure avant de calculer l'indicateur (IN), le signal de mesure étant amplifié avec un gain qui est variable en fonction du temps et augmente entre les premier et deuxième instants (t1, t2).

8. Procédé de contrôle de la stabilité d'un implant dentaire (1) inséré au moins partiellement dans un os (2), l'implant ayant une extrémité libre (1a) émergeant à la surface de l'os et une extrémité enfouie (1b) dans l'os (2), située à l'opposé de l'extrémité libre, le procédé comprenant les étapes suivantes:

- émettre une onde ultrasonore se propageant à l'intérieur de l'implant (1) vers l'extrémité enfouie (1b), recueillir l'onde ultrasonore réfléchie au niveau d'une interface de contact (4) entre l'implant (1) et l'os (2), et fournir un signal de mesure représentant l'onde ultrasonore réfléchie,
- calculer par une unité de traitement, à partir du signal de mesure, un indicateur (IN) dont la valeur permet d'évaluer l'intégration de l'implant (1) dans l'os (2), l'indicateur (IN) correspondant à l'énergie moyenne du signal de mesure sur un intervalle de temps (t1-t2) débutant à un premier instant (t1) et se terminant à un deuxième instant (t2), **caractérisé en ce que** le premier instant (t1) est compris entre 20 et 80 µs après le début de l'émission de l'onde ultrasonore par le transducteur (12).

9. Procédé selon la revendication 8, dans lequel le premier instant (t1) est compris entre 30 et 60 µs.

10. Procédé selon la revendication 8 ou 9, dans lequel le deuxième instant (t2) est, au minimum, séparé du premier instant (t1) de 5 µs et, au maximum, égal à 200 µs.

11. Procédé selon l'une quelconque des revendications 8 à 10, dans lequel l'onde ultrasonore émise a une fréquence centrale au moins égale à 5 MHz et, en particulier, au moins égale 8 MHz.

12. Procédé selon l'une quelconque des revendications 8 à 11, dans lequel, avant de calculer l'indicateur (IN), le signal de mesure est amplifié avec un gain ajusté de manière à obtenir une saturation du signal jusqu'au premier instant (t1) mais pas au delà.

13. Procédé selon l'une quelconque des revendications 8 à 12, dans lequel le gain est variable en fonction du temps et augmente entre les premier et deuxième instants (t1, t2).

**Patentansprüche**

1. Vorrichtung zum Kontrollieren der Stabilität eines Zahnimplantats (1), das mindestens teilweise in einen Knochen (2) eingesetzt ist, wobei das Implantat ein freies Ende (1a), das aus der Oberfläche des Knochens austritt, und ein im Knochen (2) vergrabenes Ende (1b) aufweist, das sich gegenüber dem freien Ende befindet, wobei die Vorrichtung Folgendes umfasst:

- einen Ultraschallwandler (12), der dazu ausgelegt ist, direkt oder indirekt mit dem Implantat (1) gekoppelt zu werden, eine Ultraschallwelle zu emittieren, die sich innerhalb des Implantats (1) in Richtung des vergrabenen Endes (1b) ausbreitet, die von einer Kontaktschnittstelle (4) zwischen dem Implantat (1) und dem Knochen (2) reflektierte Ultraschallwelle zu erfassen und ein Messsignal bereitzustellen, das die reflektierte Ultraschallwelle darstellt,
- eine Verarbeitungseinheit, die dazu ausgelegt ist, anhand des Messsignals einen Indikator (IN) zu berechnen, dessen Wert es ermöglicht, die Integration des Implantats (1) in den Knochen (2) zu bewerten, wobei der Indikator (IN) der durchschnittlichen Energie des Messsignals über ein Zeitintervall (t1-t2) entspricht, das zu einem ersten Zeitpunkt (t1) beginnt und zu einem zweiten Zeitpunkt (t2) endet, **dadurch gekennzeichnet, dass** der erste Zeitpunkt (t1) zwischen 20 und 80 µs nach dem Beginn der Emission der Ultraschallwelle durch den Wandler (12) liegt.

2. Vorrichtung nach Anspruch 1, wobei der erste Zeitpunkt (t1) zwischen 30 und 60 µs liegt.

3. Vorrichtung nach Anspruch 1 oder 2, wobei der zweite Zeitpunkt (t2) vom ersten Zeitpunkt (t1) um mindestens 5 µs und maximal 200 µs getrennt ist.

4. Vorrichtung nach einem der Ansprüche 1 bis 3, wobei der Ultraschallwandler dazu ausgelegt ist, eine Ultraschallwelle zu emittieren, deren Mittenfrequenz mindestens 5 MHz und insbesondere mindestens 8 MHz beträgt.

5. Vorrichtung nach einem der Ansprüche 1 bis 4, wobei der Ultraschallwandler (12) eine aktive Oberfläche aufweist, die dazu ausgelegt ist, direkt oder über ein Zwischenbauteil parallel zu einer durch das freie Ende des Implantats definierten Auflagefläche mit dem freien Ende (1a) des Implantats in Kontakt zu stehen, um eine Ultraschallwelle zu emittieren, die sich im Inneren des Implantats (1) in einer Ausbreitungsrichtung senkrecht zur aktiven Oberfläche des Wandlers (12) ausbreitet.

6. Vorrichtung nach einem der Ansprüche 1 bis 5, wobei die Verarbeitungseinheit Verstärkungsmittel umfasst, um das Messsignal vor dem Berechnen des Indikators (IN) zu verstärken, wobei das Messsignal mit einer Verstärkung verstärkt wird, die derart eingestellt ist, dass eine Sättigung des Signals bis zum ersten Zeitpunkt (t1), aber nicht darüber hinaus, erreicht wird.

7. Vorrichtung nach einem der Ansprüche 1 bis 6, wobei die Verarbeitungseinheit Verstärkungsmittel umfasst, um das Messsignal vor dem Berechnen des Indikators (IN) zu verstärken, wobei das Messsignal mit einer Verstärkung verstärkt wird, die als Funktion der Zeit variabel ist und zwischen dem ersten und dem zweiten Zeitpunkt (t1, t2) zunimmt.

8. Verfahren zum Kontrollieren der Stabilität eines Zahnimplantats (1), das mindestens teilweise in einen Knochen (2) eingesetzt ist, wobei das Implantat ein freies Ende (1a), das aus der Oberfläche des Knochens austritt, und ein im Knochen (2) vergrabenes Ende (1b) aufweist, das sich gegenüber dem freien Ende befindet, wobei das Verfahren die folgenden Schritte umfasst:

   - Emittieren einer Ultraschallwelle, die sich innerhalb des Implantats (1) in Richtung des vergrabenen Endes (1b) ausbreitet, Erfassen der von einer Kontaktschnittstelle (4) zwischen dem Implantat (1) und dem Knochen (2) reflektierten Ultraschallwelle und Bereitstellen eines Messsignals, das die reflektierte Ultraschallwelle darstellt,
   - Berechnen eines Indikators (IN), dessen Wert es ermöglicht, die Integration des Implantats (1) in den Knochen (2) zu bewerten, anhand des Messsignals durch eine Verarbeitungseinheit,
   wobei der Indikator (IN) der durchschnittlichen Energie des Messsignals über ein Zeitintervall (t1-t2) entspricht, das zu einem ersten Zeitpunkt (t1) beginnt und zu einem zweiten Zeitpunkt (t2) endet,
   **dadurch gekennzeichnet, dass** der erste Zeitpunkt (t1) zwischen 20 und 80 μs nach dem Beginn der Emission der Ultraschallwelle durch den Wandler (12) liegt.

9. Verfahren nach Anspruch 8, wobei der erste Zeitpunkt (t1) zwischen 30 und 60 μs liegt.

10. Verfahren nach Anspruch 8 oder 9, wobei der zweite Zeitpunkt (t2) vom ersten Zeitpunkt (t1) um mindestens 5 μs und maximal 200 μs getrennt ist.

11. Verfahren nach einem der Ansprüche 8 bis 10, wobei die emittierte Ultraschallwelle eine Mittenfrequenz von mindestens 5 MHz und insbesondere mindestens 8 MHz aufweist.

12. Verfahren nach einem der Ansprüche 8 bis 11, wobei das Messsignal vor dem Berechnen des Indikators (IN) mit einer Verstärkung verstärkt wird, die derart eingestellt ist, dass eine Sättigung des Signals bis zum ersten Zeitpunkt (t1), aber nicht darüber hinaus, erreicht wird.

13. Verfahren nach einem der Ansprüche 8 bis 12, wobei die Verstärkung als Funktion der Zeit variabel ist und zwischen dem ersten und dem zweiten Zeitpunkt (t1, t2) zunimmt.

**Claims**

1. A device for controlling the stability of a dental implant (1) inserted at least partially into a bone (2), the implant having a free end (1a) emerging at the surface of the bone and an end (1b) buried in the bone (2), located opposite the free end, the device comprising:

   - an ultrasonic transducer (12) adapted to be coupled directly or indirectly to the implant (1), to emit an ultrasonic wave propagating inside the implant (1) towards the buried end (1b), to collect the reflected ultrasonic wave at a contact interface (4) between the implant (1) and the bone (2), and to provide a measurement signal representing the reflected ultrasonic wave,
- a processing unit adapted to calculate, from the measurement signal, an indicator (IN) whose value allows to evaluate the integration of the implant (1) in the bone (2), the indicator (IN) corresponding to the average energy of the measurement signal over a time interval (t1-t2) starting at a first instant (t1) and ending at a second instant (t2), **characterised in that** the first instant (t1) is between 20 and 80 $\mu$s after the start of the emission of the ultrasonic wave by the transducer (12).

2. The device according to claim 1, wherein the first instant (t1) is comprised between 30 and 60 $\mu$s.

3. The device according to claim 1 or 2, wherein the second instant (t2) is, at a minimum, separated from the first instant (t1) by 5 $\mu$s and, at a maximum, equal to 200 $\mu$s.

4. The device according to any one of claims 1 to 3, wherein the ultrasonic transducer is adapted to emit an ultrasonic wave whose central frequency is at least equal to 5 MHz and, in particular, at least equal to 8 MHz.

5. The device according to any one of claims 1 to 4, wherein the ultrasonic transducer (12) has an active surface adapted to be in contact with the free end (1a) of the implant, directly or via an intermediate part, parallel to a support surface defined by the free end of the implant, so as to emit an ultrasonic wave propagating inside the implant (1) in a propagation direction perpendicular to the active surface of the transducer (12).

6. The device according to any one of claims 1 to 5, wherein the processing unit comprises amplification means for amplifying the measurement signal before calculating the indicator (IN), the measurement signal being amplified with a gain adjusted so as to obtain saturation of the signal up to the first instant (t1) but not beyond.

7. The device according to any one of claims 1 to 6, wherein the processing unit comprises amplification means for amplifying the measurement signal before calculating the indicator (IN), the measurement signal being amplified with a gain which is variable as a function of time and increases between the first and second instants (t1, t2).

8. A method for controlling the stability of a dental implant (1) inserted at least partially in a bone (2), the implant having a free end (1a) emerging at the surface of the bone and an end (1b) buried in the bone (2), located opposite the free end, the method comprising the following steps:

   - emitting an ultrasonic wave propagating inside the implant (1) towards the buried end (1b), collecting the reflected ultrasonic wave at a contact interface (4) between the implant (1) and the bone (2), and providing a measurement signal representing the reflected ultrasonic wave,
   - calculating by a processing unit, from the measurement signal, an indicator (IN) the value of which allows to evaluate the integration of the implant (1) in the bone (2),

   the indicator (IN) corresponding to the average energy of the measurement signal over a time interval (t1-t2) starting at a first instant (t1) and ending at a second instant (t2), **characterised in that** the first instant (t1) is comprised between 20 and 80 $\mu$s after the start of the emission of the ultrasonic wave by the transducer (12).

9. The method according to claim 8, wherein the first instant (t1) is comprised between 30 and 60 $\mu$s.

10. The method according to claim 8 or 9, wherein the second instant (t2) is, at a minimum, separated from the first instant (t1) by 5 $\mu$s and, at a maximum, equal to 200 $\mu$s.

11. The method according to any one of claims 8 to 10, wherein the emitted ultrasonic wave has a central frequency at least equal to 5 MHz and, in particular, at least equal to 8 MHz.

12. The method according to any one of claims 8 to 11, wherein, before calculating the indicator (IN), the measurement signal is amplified with a gain adjusted so as to obtain saturation of the signal up to the first instant (t1) but not beyond.

13. The method according to any one of claims 8 to 12, wherein the gain is variable as a function of time and increases between the first and second instants (t1, t2).

Fig.1

Fig.2

## FIG. 3

## FIG. 4

## FIG. 5

## FIG. 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2503954 A

**Littérature non-brevet citée dans la description**

- **ROMAIN VAYRON et al.** Assessment of In Vitro Dental Implant Primary Stability Using an Ultrasonic Method. *Ultrasound in Medicine & Biology,* vol. 40 (12), 2885-2894